# EUROPEAN PATENT SPECIFICATION

(11) **EP 3 823 950 B1**
(45) Date of publication and mention of the grant of the patent: **19.02.2025**
(21) Application number: 19759747.9
(22) Date of filing: 22.07.2019
(51) Int. Cl.: C07C 29/94, C07C 31/125

(54) **METHOD OF STORING AND/OR TRANSPORTING OXO ALCOHOL**
VERFAHREN ZUM LAGERN UND / ODER TRANSPORTIEREN VON OXOALKOHOL
PROCÉDÉ DE STOCKAGE ET/OU DE TRANSPORT D'UN OXOALCOOL

(30) Priority: 20.07.2018 US 201862700993 P
(43) Date of publication of application: 26.05.2021
(73) Proprietor: SABIC Global Technologies B.V., 4612 PX Bergen op Zoom (NL)
(72) Inventor: SOMAK, Paul, Riyadh, 11551 (SA); NANDY, Ritesh, Bangalore, Karnataka 562125 (IN); GHAMDI-AL, Ameen, Riyadh, 11422 (SA); HASYAGAR, Umesh Krishna, Bangalore, Karnataka 562125 (IN); NAIR, Vinod S., Bangalore, Karnataka 562125 (IN); AL-DUGHAITHER, Abdullah Saad, Riyadh, 11422 (SA)
(74) Representative: Balder IP Law, S.L.
(86) International application number: PCT/IB2019/056257
(87) International publication number: WO 2020/016862

(56) References cited:
- WO-A1-2006/108698
- FR-A1- 2 246 526
- GB-A- 831 592
- US-A- 2 658 923

## Description

### BACKGROUND

Oxo alcohols are prepared by adding carbon monoxide and hydrogen to an olefin to obtain an aldehyde via hydroformylation reaction. The aldehyde can then be hydrogenated to obtain the oxo alcohol. An intermediate step of adding two aldehydes together to obtain a larger aldehyde can precede the hydrogenation. Common oxo alcohols include butanol and 2-ethylhexanol (2-EH).

Oxo alcohols find use in many commercial products. For example, oxo alcohols can be used to synthesize plasticizers. Plasticizers are additives that increase the plasticity or fluidity of a material. Common plasticizers include phthalates and acrylates. The most dominant applications for plasticizers are in plastic materials, especially polyvinyl chloride (PVC). The properties of other materials are also improved when blended with plasticizers, for example, concrete, clays, and other related products. Accordingly, both oxo alcohols and plasticizers are considered commercially valuable products.

The production, storage and transport of oxo alcohols present many engineering challenges. Oxo alcohols must be maintained at high levels of purity, for example, less than 500 part per million by weight (ppm), so as not to negatively impact the quality of downstream plasticizer products. Processes such as autoxidation can occur when oxo alcohols are exposed to oxygen, resulting in the formation of unwanted aldehyde and peroxide impurities. For example, these impurities can form during the storage and/or transport of oxo alcohols and can significantly degrade plasticizer product quality. In addition, harsh weather conditions, such as temperatures in excess of 50°C, can also accelerate the autoxidation process during storage and/or transport.

The stabilization of oxo alcohols using phenolic antioxidants is known from, for example, GB831592A. However, there is a need for a method of storing and/or transporting oxo alcohols that avoids the problem of impurity formation and does not negatively affect downstream plasticizer production.

### SUMMARY

Disclosed, in various embodiments, are methods of storing and/or transporting oxo alcohol, methods of using the oxo alcohol, and methods of making the oxo alcohol.

In an embodiment, a method of storing and/or transporting oxo alcohol comprises: combining an oxo alcohol having an initial aldehyde content X' and an initial peroxide content Y' with 5 ppm to 100 ppm of a phenolic antioxidant, preferably a phenolic antioxidant without ester linkage groups; and storing and/or transporting the oxo alcohol.

These and other features and characteristics are more particularly described below.

### DETAILED DESCRIPTION

The invention provided herein is as defined in the claims.

The method disclosed herein for storing and/or transporting oxo alcohols can avoid the problem of impurity formation and does not negatively affect downstream plasticizer production. The method includes combining an oxo alcohol with a phenolic antioxidant, namely 5 ppm to 100 ppm of 1,3-tris(2'-methyl-4'-hydroxy-5'-t- butylphenyl)butane or 3,5-bis(1,1-dimethylethyl)-4-hydroxy-benzenepropanoic acid. The oxo alcohol can then be stored and/or transported for a long duration of time without compromising quality, even at temperatures in excess of 50°C (e.g., at a temperature of up to 65°C). For example, the duration of time can be greater than or equal to 3 weeks, e.g., greater than or equal to 2 months. Surprisingly, the presence of the phenolic antioxidant significantly reduces the occurrence of autoxidation, thus maintaining or even reducing impurity levels within the oxo alcohol during the storage and/or transport period. For example, aldehyde levels in the oxo alcohol can be less than or equal to 400 ppm even after 3 weeks of storage and/or transport. Even more surprisingly, the presence of the antioxidant does not negatively affect downstream plasticizer production. For example, plasticizer synthesized from the oxo alcohol can score best in class on the Platinum-Cobalt Scale in accordance with ASTM D1209, as updated in 2011.

The oxo alcohol of the present method can come from any private or commercial source. For example, the source of the oxo alcohol can be a process comprising the aldol condensation of n-butyraldehyde followed by hydrogenation of hydroxyaldehyde. The oxo alcohol can be any oxo alcohol that is suitable for the downstream production of plasticizer. For example, the oxo alcohol can comprise 2-ethylhexanol, 2-propylheptanol, isononyl alcohol, isodecyl alcohol, butanol, or a combination comprising at least one of the foregoing.

The oxo alcohol comprises an initial aldehyde content "X" and an initial peroxide content "Y". Desirably, the initial aldehyde content "X" is less than or equal to 500 ppm, preferably, less than or equal to 250 ppm, more preferably, less than or equal to 100 ppm, even more preferably, less than or equal to 50 ppm. The initial aldehyde content "X" can be 20 to 500 ppm. Desirably, the initial peroxide content "Y" is less than or equal to 15 ppm, preferably, less than or equal to 5 ppm, more preferably, less than or equal to 1 ppm. The initial peroxide content "Y" can be 0.2 to 15 ppm.

The oxo alcohol can be combined with 3,5-bis(1,1-dimethylethyl)-4-hydroxybenzenepropanoic acid as represented by Formula I, such as Anox^{™} 1315 antioxidant commercially available from Addivant^{™} Corporation.

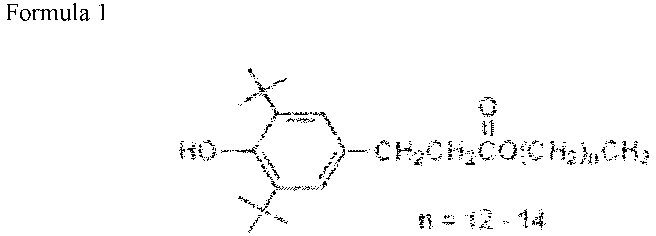

The oxo alcohol can also be combined with 1,1,3-tris(2'-methyl-4'-hydroxy-5'-t-butylphenyl)butane as represented by Formula 2, such as Lowinox^{™} CA22 antioxidant commercially available from Addivant^{™} Corporation.

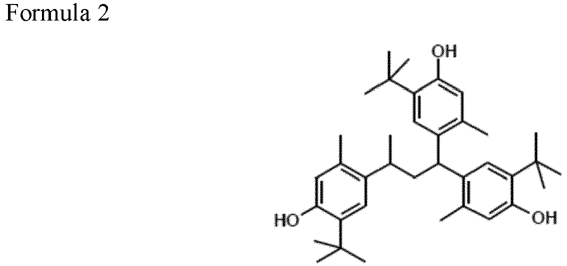

The phenolic antioxidant added to the oxo alcohol can be present in an amount of 5 ppm to 100 ppm, preferably, 5 to 80 ppm, preferably, 5 to 50 ppm, or 5 to 30 ppm, or 10 to 25 ppm. The amount of antioxidant added to the oxo alcohol can be varied in accordance with the initial aldehyde content "X" and/or the initial peroxide content "Y". For example, greater amounts of antioxidant can be added to the oxo alcohol when greater amounts of initial aldehyde content "X" and/or initial peroxide content "Y" are observed.

After combination with the antioxidant, the oxo alcohol can have a subsequent aldehyde content x and a subsequent peroxide content y. The subsequent aldehyde content x can be less than or equal to 1.10 X, preferably less than or equal to 1.00 X, or less than or equal to 0.95 X, or less than or equal to 0.80 X. The subsequent peroxide content y is less than or equal to 1.10 Y, preferably less than or equal to 1.00 Y, or less than or equal to 0.95 Y, or less than or equal to 0.80 Y.

The subsequent aldehyde content x and/or the subsequent peroxide content y can be maintained for a period of time. For example, the period can be greater than or equal to 2 weeks, preferably, greater than or equal to 3 weeks, more preferably, greater than or equal to 4 weeks, even more preferably, greater than or equal to 16 weeks. If the oxo alcohol is not combined with an antioxidant, the subsequent aldehyde content x can be greater than or equal to 2.00 X, for example, greater than or equal to 3.00 X, for example, greater than or equal to 4.00 X, after the period of time, e.g., after 2 months. If the oxo alcohol is not combined with an antioxidant, the subsequent peroxide content y can be greater than or equal to 2.00 Y, for example, greater than or equal to 3.00 Y, for example, greater than or equal to 4.00 Y, after the period of time; e.g., after 2 months.

The oxo alcohol can be stored and/or transported. For example, storage and/or transport can comprise transport via land, air, sea, or a combination comprising at least one of the foregoing. Harsh weather conditions can be experienced during storage and/or transport. For example, a temperature of the oxo alcohol can exceed 50°C, for example, 50°C to 65°C, at a point during storage and/or transport. The oxo alcohol can be exposed to oxygen during storage and/ or transport. For example, the oxo alcohol can contact atmospheric air and/or compressed air during storage and/or transport. The oxo alcohol can be stored and/or transported for a period of time. The ability of the antioxidant to reduce impurity levels in the oxo alcohol can allow for these long periods of storage and/or transport.

The oxo alcohol can be stored and/or transported within a vessel, preferably, within a storage tank, mobile tank, thermal tank, pressurized tank, atmospheric tank, transport vehicle, reservoir, cylinder, or a combination comprising at least one of the foregoing. For example, the vessel can comprise steel, concrete, glass-reinforced plastic, thermoplastic, polyethylene, or a combination comprising at least one of the foregoing.

The oxo alcohol can be used for the downstream production of plasticizer. For example, the oxo alcohol can be used to produce dioctyl phthalate, trioctyl trimellitate, acrylate, or a combination comprising at least one of the foregoing. Phthalates are esters of phthalic acid and can be used effectively as plasticizers. Phthalates can be manufactured by reacting phthalic anhydride with alcohols that range from methanol and ethanol up to tridecyl alcohol, either as a straight chain or with some branching.

Plasticizer made from the oxo alcohol of the present method can be free of unwanted discolorations, even despite the presence of antioxidant in the oxo alcohol. For example, the resulting plasticizer can have a score of less than or equal to 30 on the Platinum-Cobalt Scale, preferably, less than or equal to 25, more preferably, less than or equal to 20. The Platinum-Cobalt Scale (also known as the Apha-Hazen Scale) is a color scale/index developed as a way to evaluate pollution levels in waste water. It has since expanded to a common method of comparison of the intensity of yellow-tinted samples. It is based on dilutions of a 500 ppm platinum-cobalt solution. The American Society for Testing and Materials (ASTM) provides detailed descriptions and procedures for the "Standard Test Method for Color of Clear Liquids (Platinum-Cobalt Scale)" under designation D1209, as updated in 2011.

The following examples are merely illustrative of the methods of storing and/or transporting oxo alcohols disclosed herein and are not intended to limit the scope hereof.

### EXAMPLES

| Table 1 | | |
|---|---|---|
| Material | Name | Supplier |
| 2-ethlyhexanol | | |
| Anox^{™} 1315 | 3,5-bis(1,1-dimethylethyl)-4-hydroxy-benzenepropanoic acid | Addivant^{™} Corporation |
| Lowinox^{™} CA22 | 1,1,3-tris(2'-methyl-4'-hydroxy-5'-t- butylphenyl)butane | Addivant^{™} Corporation |
| Alkanox^{™} 240 | phenol, 2,4-bis(1,1-dimetylethyl)-phosphite (3:1) | Addivant^{™} Corporation |
| | 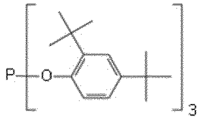 | |

### Example 1: Stability

Experimental trials were conducted in accordance with the present method. The effects of antioxidants on aldehyde impurity levels in 2-ethlyhexanol were analyzed. The results are presented in Table 2. A fixed volume of 2-ethylhexanol was admixed with an antioxidant and was heated at 50°C for three weeks in a hot metal block. The amounts and types of antioxidants are set forth in Table 2. The 50°C temperature mimicked harsh weather conditions during which accelerated autoxidation can occur. At the end of each week (intervals of 168 hours), a sample was drawn and analyzed using gas chromatography (GC). A control sample (where antioxidant was not added) was also analyzed. Lowinox^{™} CA22 was further examined at lower concentrations of 10 ppm and 20 ppm. It was surprisingly found that Lowinox^{™} CA22, even at low levels of 10 ppm, and under stressful heated conditions, was able to stop aldehyde growth for over 3 weeks.

| Table 2: Effects of Different Antioxidants on Impurity Levels in 2-Ethylhexanol | | | | | |
|---|---|---|---|---|---|
| Anox^{™} 1315 | | | | | |
| Ex. # | Amount of Antioxidant (ppm) | Aldehyde Content (ppm) | | | |
| | | 0 Hours | 168 Hours | 336 Hours | 504 Hours |
| C1 | 0 | 280 | 295 | 304 | 317 |
| E1 | 50 | 280 | 256 | 259 | 253.5 |
| E2 | 100 | 280 | 263 | 262.5 | 255.5 |
| E3 | 500 | 280 | 268 | 262.5 | 258 |

| Lowinox^{™} CA22 | | | | | |
|---|---|---|---|---|---|
| | Amount of Antioxidant (ppm) | Aldehyde Content (ppm) | | | |
| | | 0 Hours | 168 Hours | 336 Hours | 504 Hours |
| C2 | 0 | 280 | 295 | 304 | 317 |
| E4 | 50 | 280 | 266 | 259.5 | 256.5 |
| E5 | 100 | 280 | 257.5 | 254 | 250.5 |
| E6 | 500 | 280 | 256.5 | 257.5 | 248 |

| Lowinox^{™} CA22 | | | | | |
|---|---|---|---|---|---|
| | Amount of Antioxidant (ppm) | Aldehyde Content (ppm) | | | |
| | | 0 Hours | 168 Hours | 336 Hours | 504 Hours |
| C3 | 0 | 369 | 592 | 743 | 1087 |
| E7 | 10 | 369 | 317 | 311 | 319 |
| E8 | 20 | 369 | 310 | 319 | 313 |

### Example 2: Effect on Plasticizer Production

The effects of antioxidants on downstream plasticizer production were analyzed (results shown in Table 3). The oxo alcohol 2-ethylhexanol was mixed with different antioxidants (Anox^{™} 1315 and Lowinox^{™} CA22). Plasticizers dioctyl phthalate (DOP) and/or trioctyl trimellitate (TOTM) were then synthesized using the different 2-ethylhexanol mixtures. The DOP plasticizer was produced using a 2-ethylhexanol to phthalic acid molar ratio of 2.35 to 1. The TOTM plasticizer was produced using a 2-ethylhexanol to trimethylaluminium molar ratio of 3.5 to 1. The plasticizers were synthesized at a temperature of 210°C for a period of 4 hours. Phosphorous-based antioxidants, nitrogen-based antioxidants, and sulfur-based antioxidants were all found to be problematic. For example, it was observed during downstream application that when phosphorus-based antioxidants were used, initially poor solubility in the oxo alcohol was a problem and then corrosion of the reactor. Not to be limited by theory, it is believed that during the production of the plasticizers, the phosphorus-based antioxidant would be hydrolyzed, producing phosphorus acid, which corroded the reactor. Nitrogen-based antioxidants and sulfur-based antioxidants, on the other hand, adversely affected the color of the resultant plasticizers.

Color analysis of the resulting plasticizers was conducted using the Platinum-Cobalt Scale in accordance with ASTM D1209, as updated in 2011. The best in class specification for TOTM plasticizer is a score of 30 and for the DOP plasticizer is a score of 25 on the Platinum-Cobalt scale. As is shown in Table 3, trioctyl trimellitate and dioctyl phthalate were produced using 2-ethylhexanol comprising the specified antioxidant. The results are set forth in Table 3.

| Table 3 | | | | |
|---|---|---|---|---|
| | E9: TOTM | E10: DOP | E11: TOTM | E12: DOP |
| ANOX^{™} 1315 (ppm) | 50 | | | |
| Lowinox^{™} CA22 (ppm) | | 50 | 10 | 10 |

| Analysis | | | | |
|---|---|---|---|---|
| Alpha Color (Pt-Co) | 254 | 30 | 28 | 20 |

When 2-ethylhexanol with 50 ppm Anox^{™} 1315 was used, the resulting TOTM achieved a score of 254. When 2-ethylhexanol with 50 ppm Lowinox^{™} CA22 was used, the resulting TOTM achieved a score of 30. When 2-ethylhexanol with 10 ppm Lowinox^{™} CA22 was used, the resulting TOTM achieved a score of 28 and the resulting DOP achieved a score of 20. Hence, if the color is an issue due to the particular use of the plasticizer, then the phenolic antioxidant without ester linking groups can be used as it had surprisingly better color.

As is evident from these results, it was surprisingly discovered that phenolic antioxidant without an additional ester-linkage group was able to stabilize the oxo alcohol and enabled the production of a plasticizer with surprisingly good color. Surprisingly, phenolic antioxidant without an additional ester-linkage group did not affect the property or quality of the 2-ethylhexanol, nor did it interfere with properties of resulting downstream plasticizers.

### Example 3: Solubility

The solubility of different antioxidants in oxo alcohol was analyzed. Alkanox^{™} 240 (not according to the claimed invention), Lowinox^{™} CA22, and Anox^{™} 1315, were added to 2-ethylhexanol.

At a temperature of 25°C, Lowinox^{™} CA22 and Anox^{™} 1315 were surprisingly found to be soluble in 2-ethylhexanol. Alkanox^{™} 240, the phosphorus based antioxidant was not soluble. These results remained the same even when the amount of the antioxidant was varied from 100 ppm to 1,000 ppm.

The endpoints of all ranges directed to the same component or property are inclusive and independently combinable (e.g., ranges of "less than or equal to 25 wt%, or 5 wt% to 20 wt%," is inclusive of the endpoints and all intermediate values of the ranges of "5 wt% to 25 wt%," etc.). Disclosure of a narrower range or more specific group in addition to a broader range is not a disclaimer of the broader range or larger group. "Combination" is inclusive of blends, mixtures, alloys, reaction products, and the like. Furthermore, the terms "first," "second," and the like, herein do not denote any order, quantity, or importance, but rather are used to denote one element from another. The terms "a" and "an" and "the" herein do not denote a limitation of quantity, and are to be construed to cover both the singular and the plural, unless otherwise indicated herein or clearly contradicted by context. "Or" means "and/or." Reference throughout the specification to "one embodiment", "another embodiment", "an embodiment", and so forth, means that a particular element (e.g., feature, structure, and/or characteristic) described in connection with the embodiment is included in at least one embodiment described herein, and may or may not be present in other embodiments. In addition, it is to be understood that the described elements may be combined in any suitable manner in the various embodiments.

"Optional" or "optionally" means that the subsequently described event or circumstance can or cannot occur, and that the description includes instances where the event occurs and instances where it does not. Unless defined otherwise, technical and scientific terms used herein have the same meaning as is commonly understood by one of skill in the art to which this invention belongs.

## Claims

1. A method of storing and/or transporting oxo alcohol, comprising:
combining an oxo alcohol having an initial aldehyde content X and an initial peroxide content Y with 5 ppm to 100 ppm of a phenolic antioxidants; and
storing and/or transporting the oxo alcohol;
wherein the phenolic antioxidant is 1,1,3-tris(2'-methyl-4'-hydroxy-5'-t-butylphenyl)butane or 3,5-bis(1,1-dimethylethyl)-4-hydroxy-benzenepropanoic acid.

2. The method of Claim 1, wherein after a period of time, the oxo alcohol with the antioxidant has a subsequent aldehyde content "x" and a subsequent peroxide content "y" after a period of time;
wherein the period of time is greater than or equal to 2 weeks, preferably greater than or equal to 3 weeks, more preferably greater than or equal to 4 weeks, or greater than or equal to 16 weeks; and
wherein the subsequent aldehyde content x is less than or equal to 1.10 X, preferably less than or equal to 1.00 X, or less than or equal to 0.95 X, or less than or equal to 0.80 X; and/or
wherein the subsequent peroxide content y is less than or equal to 1.10 Y, preferably less than or equal to 1.00 Y, or less than or equal to 0.95 Y, or less than or equal to 0.80 Y.

3. The method of any of the preceding claims, wherein the oxo alcohol comprises 2-ethylhexanol, 2-propylheptanol, isononyl alcohol, isodecyl alcohol, butanol, or a combination comprising at least one of the foregoing.

4. The method of any of the preceding claims, wherein the amount of the phenolic antioxidant is 5 to 80 ppm, preferably, 5 to 50 ppm, or 5 to 30 ppm.

5. The method of any of the preceding claims, wherein a temperature of the oxo alcohol during the storage and/or transport is greater than or equal to 40°C, or greater than or equal to 50°C, or greater than or equal to 65°C.

6. The method of any of the preceding claims, wherein the oxo alcohol contacts oxygen during the storage and/or transport.

7. The method of any of the preceding claims, wherein autoxidation of the oxo alcohol does not occur during storage and/or transport.

8. The method of any of the preceding claims, wherein the initial aldehyde content X is greater than or equal to 20 ppm.

9. The method of any of the preceding claims, wherein the initial peroxide content Y is greater than or equal to 0.2 ppm.

10. A method of producing a plasticizer, comprising:
esterification of an acid and/or an anhydride with an oxo alcohol having an initial aldehyde content X and an initial peroxide content Y with 5 ppm to 100 ppm of a phenolic antioxidants, wherein the phenolic antioxidant is 1,1,3-tris(2'-methyl-4'-hydroxy-5'-t-butylphenyl)butane or 3,5-bis(1,1-dimethylethyl)-4-hydroxy-benzenepropanoic acid, to produce the plasticizer; and
isolating, and preferably purifying, the plasticizer.

11. The method of Claim 10, wherein the plasticizer comprises dioctyl phthalate, trioctyl trimellitate, acrylate, or a combination comprising at least one of the foregoing.

12. The method of any of Claims 10 or 11, wherein the plasticizer has a score of less than or equal to best in class on the Platinum-Cobalt Scale according to ASTM D1209, as updated in 2011, preferably, less than best in class.

## Patentansprüche

1. Verfahren zur Lagerung und/oder zum Transport von Oxoalkohol, das Folgendes umfasst:
Kombinieren eines Oxoalkohols mit einem anfänglichen Aldehydgehalt X und einem anfänglichen Peroxidgehalt Y mit 5 ppm bis 100 ppm eines phenolischen Antioxidans; und
Lagern und/oder Transportieren des Oxoalkohols;
wobei das phenolische Antioxidans 1,1,3-Tris(2'-methyl-4'-hydroxy-5'-t-butylphenyl)butan oder 3,5-Bis(1,1-dimethylethyl)-4-hydroxybenzolpropansäure ist.

2. Verfahren nach Anspruch 1, wobei der Oxoalkohol mit dem Antioxidans nach einem Zeitraum einen folgenden Aldehydgehalt "x" und nach einem Zeitraum einen folgenden Peroxidgehalt "y" aufweist;
wobei der Zeitraum größer oder gleich 2 Wochen beträgt, vorzugsweise größer oder gleich 3 Wochen, bevorzugter größer oder gleich 4 Wochen oder größer oder gleich 16 Wochen; und
wobei der folgende Aldehydgehalt x kleiner oder gleich 1,10 X ist, vorzugsweise kleiner oder gleich 1,00 X, oder kleiner oder gleich 0,95 X, oder kleiner oder gleich 0,80 X; und/oder
wobei der folgende Peroxidgehalt y kleiner oder gleich 1,10 Y ist, vorzugsweise kleiner oder gleich 1,00 Y, oder kleiner oder gleich 0,95 Y, oder kleiner oder gleich 0,80 Y.

3. Verfahren nach einem der vorherigen Ansprüche, wobei der Oxoalkohol 2-Ethylhexanol, 2-Propylheptanol, Isononylalkohol, Isodecylalkohol, Butanol oder eine Kombination aus mindestens einem der Vorgenannten umfasst.

4. Verfahren nach einem der vorherigen Ansprüche, wobei die Menge des phenolischen Antioxidans 5 bis 80 ppm, vorzugsweise 5 bis 50 ppm oder 5 bis 30 ppm beträgt.

5. Verfahren nach einem der vorherigen Ansprüche, wobei die Temperatur des Oxoalkohols während der Lagerung und/oder des Transports größer oder gleich 40 °C beträgt, oder größer oder gleich 50 °C oder größer oder gleich 65 °C.

6. Verfahren nach einem der vorherigen Ansprüche, wobei der Oxoalkohol während der Lagerung und/oder des Transports mit Sauerstoff in Kontakt kommt.

7. Verfahren nach einem der vorherigen Ansprüche, wobei während der Lagerung und/oder des Transports keine Autoxidation des Oxoalkohols stattfindet.

8. Verfahren nach einem der vorherigen Ansprüche, wobei der anfängliche Aldehydgehalt X größer oder gleich 20 ppm ist.

9. Verfahren nach einem der vorherigen Ansprüche, wobei der anfängliche Peroxidgehalt Y größer oder gleich 0,2 ppm ist.

10. Verfahren zur Herstellung eines Weichmachers, das Folgendes umfasst:
Veresterung einer Säure und/oder eines Anhydrids mit einem Oxoalkohol mit einem anfänglichen Aldehydgehalt X und einem anfänglichen Peroxidgehalt Y mit 5 ppm bis 100 ppm eines phenolischen Antioxidans, wobei das phenolische Antioxidans 1,1,3-Tris(2'-methyl-4'-hydroxy-5'-t-butylphenyl)butan oder 3,5-Bis(1,1-dimethylethyl)-4-hydroxy-benzolpropansäure ist, um den Weichmacher herzustellen; und
Isolieren und vorzugsweise Reinigen des Weichmachers.

11. Verfahren nach Anspruch 10, wobei der Weichmacher Dioctylphthalat, Trioctyltrimellitat, Acrylat oder eine Kombination aus mindestens einem der Vorgenannten umfasst.

12. Verfahren nach einem der Ansprüche 10 oder 11, wobei der Weichmacher einen Wert von weniger als oder gleich dem Bestwert seiner Klasse auf der Platin-Kobalt-Skala gemäß ASTM D1209 aufweist, wie aktualisiert im Jahr 2011, vorzugsweise von weniger als dem Bestwert.

## Revendications

1. Méthode de stockage et/ou de transport d'un oxoalcool, comprenant :
la combinaison d'un oxoalcool ayant une teneur initiale en aldéhyde X et une teneur initiale en peroxyde Y avec 5 ppm à 100 ppm d'un antioxydant phénolique ; et
le stockage et/ou le transport de l'oxoalcool ;
dans laquelle l'antioxydant phénolique est le 1,1,3-tris(2'-méthyl-4'-hydroxy-5'-t-butylphényl)butane ou l'acide 3,5-bis(1,1-diméthyléthyl)-4-hydroxybenzènepropanoïque.

2. Méthode selon la revendication 1, dans laquelle, après une certaine période de temps, l'oxoalcool avec l'antioxydant a une teneur subséquente en aldéhyde "x" et une teneur subséquente en peroxyde "y" après une certaine période de temps ;
dans laquelle la certaine période de temps est supérieure ou égale à 2 semaines, de préférence supérieure ou égale à 3 semaines, de manière plus préférée supérieure ou égale à 4 semaines, ou supérieure ou égale à 16 semaines ; et
dans laquelle la teneur subséquente en aldéhyde x est inférieure ou égale à 1,10 X, de préférence inférieure ou égale à 1,00 X, ou inférieure ou égale à 0,95 X, ou inférieure ou égale à 0,80 X ; et/ou
dans laquelle la teneur subséquente en peroxyde y est inférieure ou égale à 1,10 Y, de préférence inférieure ou égale à 1,00 Y, ou inférieure ou égale à 0,95 Y, ou inférieure ou égale à 0,80 Y.

3. Méthode selon l'une quelconque des revendications précédentes, dans laquelle l'oxoalcool comprend le 2-éthylhexanol, le 2-propylheptanol, l'alcool isononylique, l'alcool isodécylique, le butanol, ou une combinaison comprenant au moins l'un des précédents.

4. Méthode selon l'une quelconque des revendications précédentes, dans laquelle la quantité de l'antioxydant phénolique est de 5 à 80 ppm, de préférence de 5 à 50 ppm, ou de 5 à 30 ppm.

5. Méthode selon l'une quelconque des revendications précédentes, dans laquelle la température de l'oxoalcool pendant le stockage et/ou le transport est supérieure ou égale à 40 °C, ou supérieure ou égale à 50 °C, ou supérieure ou égale à 65 °C.

6. Méthode selon l'une quelconque des revendications précédentes, dans laquelle l'oxoalcool vient en contact avec de l'oxygène pendant le stockage et/ou le transport.

7. Méthode selon l'une quelconque des revendications précédentes, dans laquelle il ne se produit pas d'auto-oxydation de l'oxoalcool pendant le stockage et/ou le transport.

8. Méthode selon l'une quelconque des revendications précédentes, dans laquelle la teneur initiale en aldéhyde X est supérieure ou égale à 20 ppm.

9. Méthode selon l'une quelconque des revendications précédentes, dans laquelle la teneur initiale en peroxyde Y est supérieure ou égale à 0,2 ppm.

10. Méthode de production d'un plastifiant, comprenant :
l'estérification d'un acide et/ou d'un anhydride avec un oxoalcool ayant une teneur initiale en aldéhyde X et une teneur initiale en peroxyde Y avec 5 ppm à 100 ppm d'un antioxydant phénolique, dans laquelle l'antioxydant phénolique est le 1,1,3-tris(2'-méthyl-4'-hydroxy-5'-t-butylphényl)butane ou l'acide 3,5-bis(1,1-diméthyléthyl)-4-hydroxybenzènepropanoïque, pour produire le plastifiant ; et
l'isolation, et de préférence la purification, du plastifiant.

11. Méthode selon la revendication 10, dans laquelle le plastifiant comprend le phtalate de dioctyle, le trimellitate de trioctyle, un acrylate, ou une combinaison comprenant au moins l'un des précédents.

12. Méthode selon l'une quelconque des revendications 10 et 11, dans laquelle le plastifiant a un score inférieur ou égal au meilleur de sa catégorie sur l'échelle platine-cobalt conformément à la norme ASTM D1209, telle que mise à jour en 2011, de préférence inférieur au meilleur de sa catégorie.
